**Europäisches Patentamt**

**European Patent Office**

**Office européen des brevets**

(11) Veröffentlichungsnummer: **0 388 881**
**A1**

## (12) EUROPÄISCHE PATENTANMELDUNG

(21) Anmeldenummer: **90105223.3**

(22) Anmeldetag: **20.03.90**

(51) Int. Cl.⁵: **A61F 2/28**

(30) Priorität: **21.03.89 DE 3909182**

(43) Veröffentlichungstag der Anmeldung:
**26.09.90 Patentblatt 90/39**

(84) Benannte Vertragsstaaten:
**AT BE CH DE DK ES FR GB GR IT LI LU NL SE**

(71) Anmelder: **BRISTOL-MYERS SQUIBB COMPANY**
**345 Park Avenue**
**New York, N.Y. 10154(US)**

(72) Erfinder: **Schelhas, Klaus-Dieter**
**Leerkämpe 12**
**D-2800 Bremen 66(DE)**

(74) Vertreter: **Eisenführ, Speiser & Strasse**
**Martinistrasse 24**
**D-2800 Bremen 1(DE)**

(54) **Anordnung zum Verbinden zweier stabförmiger Prothesenteile.**

(57) Es wird eine Anordnung zum Verbinden zweier stabförmiger Prothesenteile (2,12) angegeben, die eine Spannhülse (20) aufweist, welche an ihren Enden gegenläufige Innengewinde (22,24) besitzt, die mit entsprechenden Außengewinden (4,14) der Prothesenteile verschraubbar sind. Um ein Lösen der Verbindung zu verhindern, sind die freien Enden der Prothesenteile mit komplementären schrägen Anlageflächen (8,18) versehen, die beim Spannen der Spannhülse (20) einen belasteten Schrägstoß bilden und daher die Verschraubungen unter Last setzen.

EP 0 388 881 A1

Die Erfindung betrifft eine Anordnung zum Verbinden zweier stabförmiger Prothesenteile, mit einer Spannhülse, die an ihren Enden gegenläufige Innengewinde aufweist, welche mit entsprechenden Außengewinden der Prothesenteile verschraubbar sind.

Derartige Anordnungen sind bekannt und dienen dazu, aus mehreren Teilen zusammengesetzte Prothesen, z.B. Prothesenschäfte oder Totalprothesen des menschlichen Femur, miteinander zu verbinden. Derartige Anordnungen werden außerdem eingesetzt, um bei bestimmten Indikationen den Schaft einer Knieprothese oder einer Hüftprothese an einem Knochennagel zu verankern, der zuvor in den Knochenmarkraum des betreffenden menschlichen Knochens eingesetzt worden ist. Neben der gewünschten Zug- und Druckfestigkeit muß dabei die Anordnung insbesondere auch gewährleisten, daß die beiden Prothesenteile rotationsfest, d.h. unverdrehbar miteinander verbunden sind. Gerade die drehfeste, d.h. unverdrehbare Verkopplung der beiden Prothesenteile stellt dabei ein wesentliches Problem dar, welches bei den bekannten Anordnungen unter Verwendung zusätzlicher Sicherungsmittel, wie Sicherungsschrauben etc., nur unvollkommen gelöst wird.

Aufgabe der Erfindung ist es daher, die Anordnung der eingangs genannten Art derart weiterzubilden, daß eine besonders einfache und dabei drehfeste Verbindung der Prothesenteile gewährleistet ist.

Diese Aufgabe wird bei der Anordnung der eingangs genannten Art erfindungsgemäß dadurch gelöst, daß die freien Enden der Prothesenteile schräge Anlageflächen aufweisen, die beim Spannen der Spannhülse einen Schrägstoß bilden und daher aneinander abgleiten.

Die Vorteile der Erfindung liegen insbesondere darin, daß die freien Enden der Prothesenteile schräge Anlageflächen aufweisen, die gegeneinander anlaufen und einen Schrägstoß bilden, wenn die Spannhülse mit ihren endseitigen, gegenläufigen Innengewinden mit den entsprechenden Außengewinden der Prothesenteile verschraubt wird und dabei die Prothesenteile zusammenzieht. Dabei verschieben sich die schrägen Anlageflächen gegeneinander über einen gewünschten Spannweg hinweg und erzeugen in Längsrichtung wirkende Gegenkräfte, welche die Schraubverbindung zwischen Spannhülse und den Prothesenteilen unter Last setzen und daher das unerwünschte Lockern der Schraubverbindung verhindern. Auf diese Weise wird eine selbsthemmende Verschraubung zwischen den Prothesenteilen und der Spannhülse erzeugt, ohne daß zusätzliche Sicherungsmittel, wie Querstifte oder Sicherungsschrauben benötigt werden, die ebenfalls der Gefahr einer Lockerung unterliegen. Dadurch, daß die schrägen Anlageflächen gegeneinander zur Anlage kommen, wird außerdem eine Verdrehbarkeit der Prothesenteile relativ zueinander zuverlässig verhindert, weil die schrägen Anlageflächen keine Rotationssymmetrie zur Längsachse der Prothesenteile besitzen.

Besonders bevorzugt sind die schrägen Anlageflächen der beiden Prothesenteile komplementär zueinander, d.h. sie besitzen denselben Neigungswinkel gegenüber der Längsachse der Prothesenteile und kommen flächig zur Anlage aneinander, wenn die Verbindung gespannt wird.

Gemäß einer bevorzugten Ausführungsform der Erfindung sind die freien Enden der Prothesenteile als Zylinderzapfen mit einem reduzierten Querschnitt ausgebildet, und die schrägen Anlageflächen sind an den Zylinderzapfen an ihren freien Stirnseiten angeformt. Die schrägen Anlageflächen werden bevorzugt von einer Ebene gebildet, welche die Zylinderzapfen unter einem vorgegebenen Winkel zur Zapfen-Längsachse schneidet. Je nach Wahl dieses Schnittwinkels ist dabei die auf die Verschraubung wirkende Gegenkraft unterschiedlich, welche ein Lösen der Spannhülse hemmt.

Besonders bevorzugt besitzt die Spannhülse einen Innendurchmesser, der nur geringfügig größer ist als der Durchmesser der Zylinderzapfen der beiden Prothesenteile. Wenn dann die beiden schrägen Anlageflächen gegeneinander in Längsrichtung verschoben werden, werden die Zylinderzapfen gleichzeitig quer zur Längsrichtung nach außen gegen die Innenfläche der Spannhülse gedrängt und kommen kraftschlüssig an der Innenfläche der Spannhülse zur Anlage. Dadurch wird die Verbindung der beiden Prothesenteile zusätzlich in der Spannhülse verspannt.

Gemäß einer weiteren bevorzugten Ausführungsform der Erfindung besitzt die Verschraubung am einen Ende der Spannhülse eine andere Gewindesteigung als die Verschraubung am anderen Ende der Spannhülse, wobei insbesondere eine Verschraubung als Feingewinde ausgebildet ist, während die andere Verschraubung ein Normalgewinde darstellt. Diese Ausführungsform der Erfindung besitzt den Vorteil, daß die Hemmkraft des Feingewindes beim Verspannen der Verbindung besonders groß ist und auch bei relativ geringem Spannweg über mehrere Gewindegänge hinweg wirkt, wohingegen das Normalgewinde mit seiner entsprechenden Gewindesteigung ein Verschrauben der Spannhülse mit wenigen Umdrehungen gestattet.

Vorteilhafte Weiterbildungen der Erfindung sind durch die Merkmale der Unteransprüche gekennzeichnet.

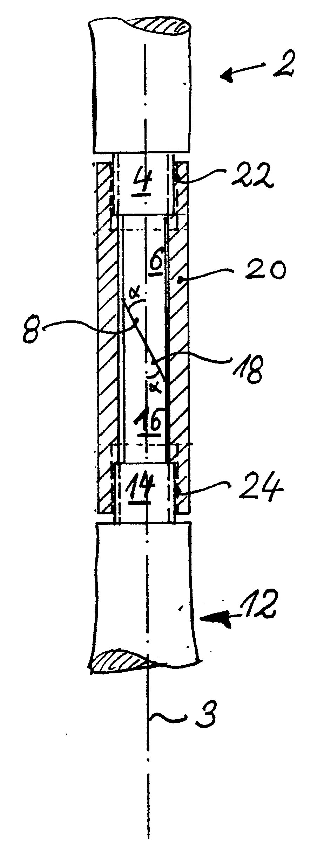

Im folgenden wird ein Ausführungsbeispiel der Erfindung anhand der Zeichnung näher erläutert.

Die Figur zeigt einen Querschnitt durch eine erfindungsgemäße Anordnung.

Ein erstes stabförmiges Prothesenteil 2 besitzt an seinem freien Ende einen Gewindezapfen 6, dessen endseitige Stirnfläche als schräge Anlagefläche 8 ausgebildet ist. Die schräge Anlagefläche 8 ist eine Ebene, welche die Längsachse 3 des Prothesenteils 2 unter einem spitzen Winkel α schneidet. Ein zweites stabförmiges Prothesenteil 12 ist an seinem freien Ende ebenfalls als Zylinderzapfen 16 ausgebildet, dessen freie Stirnseite ebenfalls als schräge Anlagefläche 18 ausgebildet ist. Die Anlagefläche 18 ist ebenfalls eben und schneidet die Längsachse 3 der beiden Prothesenteile 2, 12 unter dem Winkel α. In vorgegebenem Abstand von den schrägen Anlageflächen 8, 18 tragen die Zylinderzapfen 6, 16 je ein Außengewinde 4, 14.

Eine Spannhülse 20 umgibt die Zylinderzapfen 6, 16 und besitzt an ihren Enden gegenläufige Innengewinde 22, 24, und ist mittels der Innengewinde 22, 24 mit den entsprechenden Außengewinden 4, 14 der Prothesenteile 2, 12 verschraubbar. Die Zapfen 6, 16 besitzen eine solche Länge, daß die schrägen Anlageflächen 8, 18 gegeneinander zur Anlage kommen, wenn die Spannhülse 20 mit den Außengewinden 4, 14 der Prothesenteile 2, 12 verschraubt wird. Der Innendurchmesser der Spannhülse 20 ist nur geringfügig größer als der Durchmesser der Zylinderzapfen 6, 16. Die Gewinde 4, 14 besitzen in der dargestellten Ausführungsform gegenüber dem Zylinderzapfen einen geringfügig größeren und gegenüber dem dann folgenden Abschnitt des betreffenden Prothesenteils 2, 12 einen reduzierten Durchmesser. Der Außendurchmesser der Spannhülse 20 entspricht etwa dem Durchmesser, den die Prothesenteile im Anschluß an die Außengewinde 4, 14 besitzen, so daß der Außendurchmesser über die gesamte Länge des Verbundes etwa konstant bleibt.

Das Außengewinde eines Prothesenteils und das entsprechende Innengewinde der Spannhülse 20 sind als Feingewinde ausgebildet. Das Außengewinde des anderen Prothesenteils und das zugehörige andere Innengewinde der Spannhülse 20 sind bevorzugt als Normalgewinde ausgebildet.

Wenn die Spannhülse 20 mit den Prothesenteilen 2, 12 verschraubt wird, gelangen die schrägen Anlageflächen 8, 18 - bei entsprechender Ausrichtung der Prothesenteile - gegeneinander zur Anlage und bilden dann einen Schrägstoß. Wird dann die Spannhülse 20 weiter auf die Außengewinde 4, 14 aufgeschraubt, so verschieben sich die Anlageflächen 8, 18 gegeneinander und werden dabei quer zur Längsachse 3 nach außen gegen die Innenfläche der Spannhülse gepreßt. Außerdem werden beim Gegeneinanderlaufen der Anlageflächen 8, 18 in Achsrichtung gerichtete Gegenkräfte erzeugt, welche die Verschraubung belasten und ein Lösen der Spannhülse 20 insbesondere beim Feingewinde hemmen.

Die Relativverschiebung, welche die Anlageflächen 8, 18 in Längsrichtung noch zurücklegen, nachdem die Anlageflächen 8, 18 in Berührungskontakt gelangt sind, wird als Spannweg bezeichnet. Je nach Neigung der schrägen Anlageflächen 8, 18 kann ein relativ großer Spannweg verwirklicht werden, der über mehrere Umdrehungen der Spannhülse 20 hinweg die Verschraubung mit der Gegenkraft oder Spannkraft beaufschlagt, welche die Verschraubung unter Last hält und damit am Lösen hindert. Die Verschraubung ist dadurch - ggfs. über mehrere Gewindegänge hinweg - gegen eine Lockerung gesichert.

## Ansprüche

1. Anordnung zum Verbinden zweier stabförmiger Prothesenteile, mit einer Spannhülse, die an ihren Enden gegenläufige Innengewinde aufweist, welche mit entsprechenden Außengewinden der Prothesenteile verschraubbar sind, dadurch gekennzeichnet, daß die freien Enden (6, 16) der Prothesenteile (2, 12) schräge Anlageflächen (8, 18) aufweisen, die beim Spannen der Spannhülse (20) aneinander abgleiten.

2. Anordnung nach Anspruch 1, dadurch gekennzeichnet, daß die freien Enden (6, 16) der Prothesenteile (2, 12) als Zylinderzapfen ausgebildet sind, daß die schrägen Anlageflächen (8, 18) an den Zylinderzapfen stirnseitig angeformt sind, und daß der Innendurchmesser der Spannhülse (20) nur geringfügig größer ist als der Durchmesser der Zylinderzapfen (6, 16).

3. Anordnung nach Anspruch 1 oder 2, dadurch gekennzeichnet, daß die an dem einen Prothesenteil zusammenwirkenden Gewinde (4, 22) eine andere Steigung besitzen, als die am anderen Prothesenteil (12) zusammenwirkenden Gewinde (14, 24).

4. Anordnung nach einem der vorstehenden Ansprüche, dadurch gekennzeichnet, daß die Gewinde (4, 14) gegenüber dem benachbarten Abschnitt der Prothesenteile (2, 12) einen reduzierten Durchmesser besitzen, und daß der Außendurchmesser der Spannhülse (20) etwa dem Durchmesser der benachbarten Abschnitte der Prothesenteile (2, 12) entspricht.

5. Anordnung nach einem der vorstehenden Ansprüche, dadurch gekennzeichnet, daß auf der Außenfläche der Spannhülse (20) ein Mehrkant zum Ansetzen eines Spannwerkzeuges

angeformt ist.

| | **EINSCHLÄGIGE DOKUMENTE** | | EP 90105223.3 |
|---|---|---|---|
| Kategorie | Kennzeichnung des Dokuments mit Angabe, soweit erforderlich. der maßgeblichen Teile | Betrifft Anspruch | KLASSIFIKATION DER ANMELDUNG (Int. Cl⁵) |
| A | <u>DE - C2 - 3 605 630</u><br>(ORTHOPLANT ENDOPROTHETIK GMBH)<br>    * Spalte 4, Zeilen 6-20, 31-42; Spalte 5, Zeilen 13-15; Figuren 1,2 *<br>-- | 1,4,5 | A 61 F 2/28 |
| A | <u>DE - C3 - 2 605 180</u>  . .<br>(TALLINSKIJ POLITECHNIT-SCHESKIJ INSTITUT)<br>    * Spalte 5, Zeile 13 - Spalte 6, Zeile 2; Figuren 3,4 *<br>---- | 1,5 | |
| | | | RECHERCHIERTE SACHGEBIETE (Int. Cl⁵) |
| | | | A 61 F 2/00 |

Der vorliegende Recherchenbericht wurde für alle Patentansprüche erstellt.

| Recherchenort | Abschlußdatum der Recherche | Prüfer |
|---|---|---|
| WIEN | 14-05-1990 | VELINSKY-HUB |

EPA Form 1503 03 82